# EUROPÄISCHE PATENTSCHRIFT

(11) **EP 0 710 469 B1**
(45) Veröffentlichungstag und Bekanntmachung des Hinweises auf die Patenterteilung: **18.12.2002**
(21) Anmeldenummer: 95116666.9
(22) Anmeldetag: 23.10.1995
(51) Int. Cl.: A61F 9/00

(54) **Vorrichtung zum Zerkleinern und Entfernen des Linsenkerns**
Device for the comminution and removal of a lens core
Dispositif de désintégration et d'enlèvement du noyau du cristallin

(30) Priorität: 07.11.1994 CH 331794
(43) Veröffentlichungstag der Anmeldung: 08.05.1996
(73) Patentinhaber: GRIESHABER & CO. AG SCHAFFHAUSEN, CH-8203 Schaffhausen (CH)
(72) Erfinder: Grieshaber, Hans R., CH-8200 Schaffhausen (CH); Stegmann, Robert, Prof. M.D., Pretoria, 0181 (ZA)
(74) Vertreter: Althoff, Gerhard

(56) Entgegenhaltungen:
- EP-A- 0 623 328
- WO-A-94/21183
- DE-A- 2 035 181
- US-A- 3 937 222
- US-A- 4 368 734
- US-A- 4 753 234

## Beschreibung

Die Erfindung bezieht sich auf eine Vorrichtung zum Zerkleinern und Entfernen des Linsenkerns aus dem Auge eines Lebewesens, mit einem als Handstück ausgebildeten Gehäuse, einer daran angeordneten Halterung zur Aufnahme eines mit einer Infusionsleitung in Verbindung stehenden Führungsrohres, einer koaxial darin angeordneten und relativ dazu um eine gemeinsame Längsachse rotierend und/oder in axialer Richtung oszillierend antreibbaren Rohrsonde, welche an dem einen Ende mit einer Saugleitung verbunden und an dem anderen Ende mit einem teilweise aus dem Führungsrohr ragenden Kopfstück und daran angeordnetem Schneidelement versehen ist.

Die Linse (Okular) bildet in Verbindung mit den Zonularfasern und dem Ziliarmuskel einen Teil des optischen Augensystems, wobei in Abhängigkeit von der Beschaffenheit (Flexibilität) des Linsenkerns die Einstellung (Akkomodation) der Brechkraft erfolgt. Durch die als sogenannter Grauer Star (Katarakt) bekannte Eintrübung und/oder Verhärtung des Linsenkerns wird das Sehvermögen erheblich beeinträchtigt.

Zum Zerkleinern und Entfernen des Linsenkerns aus dem Auge eines Lebewesens ist aus der EP-A 0 623 328 eine Vorrichtung bekannt, welche ein als Handstück ausgebildetes Gehäuse mit darin angeordneten Antriebsmitteln, ein in dem Gehäuse gelagertes Führungsrohr, eine koaxial darin angeordnete und relativ dazu um eine gemeinsame Längsachse rotierend und/oder in axialer Richtung oszillierend antreibbare Rohrsonde, ein am vorderen Ende derselben angeordnetes sowie über eine Ansaugöffnung mit dem Innenraum der Rohrsonde in Verbindung stehendes und etwa trichterförmig ausgebildetes Kopfstück umfasst, welches mit zwei seitlich gegenüberliegend zueinander angeordneten und als Flügelstücke ausgebildeten Schneidelementen versehen ist.

Eine ähnliche Vorrichtung gemäß dem Oberbegriff des Anspruchs 1 ist weiterhin aus der WO-94/21183 bekannt, welche ein zur Aufnahme eines Antriebs ausgebildetes Gehäuse, eine darin gelagerte äussere Hülse, ein koaxial darin angeordnetes Führungsrohr für die Irrigation sowie eine koaxial darin angeordnete und mit dem Antrieb wirkverbundene Rohrsonde für die Aspiration umfasst. An dem aus dem Führungsrohr ragenden Teilstück der Rohrsonde ist ein in axialer Richtung orientiertes Schneidelement angeordnet, welches ausgehend von einem etwa haarnadelförmig umgebogenen Scheitelpunkt (Apex) mit zwei in Bezug auf die theoretische Mittelachse etwa schlaufenförmig nach aussen gebogenen und jeweils mit einer angeschliffenen Profilkante versehene Schenkel aufweist, welche im Bereich der Rohrsonde jeweils bis etwa zur Mitte der Eintrittsöffnung nach innen gebogen an der Rohrsonde befestigt sind.

Aus der US-A 3,990,453 ist eine Vorrichtung zum Entfernen des Grauen Starts (Katarakt) bekannt, welche ein zur Aufnahme eines Antriebsmotors ausgebildetes Gehäuse, ein daran befestigtes Kopfstück, eine darin angeordnete und mit einer Saugpumpe in Verbindung stehende Hohlnadel umfasst, welche an der Spitze als sägezahnförmiges Schneidelement ausgebildet ist und von dem Antriebsmotor rotierend angetrieben wird.

Der Erfindung liegt die Aufgabe zugrunde, eine Vorrichtung gemäss der im Oberbegriff des Patentanspruchs 1 genannten Gattung dahingehend zu verbessern, dass auch extrem harte Linsenkerne zerkleinert und etwa in Form einer breiigen Masse abgesaugt werden können, ohne dabei die hochempfindlichen inneren Zonen (Kapselhaut) des Kapselsacks zu verletzen.

Die erfindungsgemässe Vorrichtung ist dadurch gekennzeichnet, dass das Kopfstück mit einem ersten zylindrischen Teilstück, einem daran angeformten konischen Teilstück sowie einem daran angeformten zweiten zylindrischen Teilstück etwa trichterförmig ausgebildet und über eine Ansaugöffnung mit dem Innenraum der Rohrsonde verbunden ist, dass das Schneidelement mit einem Bogenteil und daran angeordneter Schneidkante die Ansaugöffnung des Kopfstücks etwa kreisbogenförmig überspannt sowie mit zwei achsparallel zur Längsachse angeformten Schenkeln versehen ist, dass das Schneidelement mittels der beiden Schenkel derart an dem ersten zylindrischen Teilstück des Kopfstücks angeordnet und befestigt ist, dass ausgehend von der die Ansaugöffnung des zweiten zylindrischen Teilstücks kreisringförmig umgebenden Fläche der bis zur Innenseite des Bogenteils in axialer Richtung orientierte Abstand kleiner als der quer dazu über die beiden Schenkel des Bogenteils gemessene äussere Abstand ist.

Die erfindungsgemässe Vorrichtung hat den Vorteil, dass unter Beibehaltung der relativ kleinen und in der Grössenordnung von etwa 1,6 mm liegenden äusseren Abmessung des am Kopfstück angeordneten Schneidelements dieses auch bei der relativ hohen Rotations- und Oszillationsbewegung eine ausreichende Stabilität aufweist und somit zum Zerkleinern und Emulsifizieren des verhärteten Linsenkerns exakt im Inneren der Linse geführt werden kann ohne dabei infolge einer etwaigen Taumelbewegung den hochempfindlichen Kapselsack zu verletzen. Ein weiterer Vorteil besteht darin, dass die von dem Schneidelement abgetrennten Linsenpartikel infolge der etwa im Bereich der Schneidkante wirkenden Saugleistung ohne Aufwirbelung direkt in die Ansaugöffnung des Kopfstücks gesaugt und über den Innenraum der angeschlossenen Rohrsonde abgeführt werden. Zur Erreichung der unmittelbar im Bereich der Schneidkante wirkenden Saugleistung ist der zwischen der kreisringförmigen Fläche des zweiten zylindrischen Teilstücks und der bogenförmigen Innenkante des Schneidelements in axialer Richtung orientierte Abstand kleiner als der quer dazu über die beiden Schenkel des Bogenteils gemessene und etwa 1,6 mm betragende äussere Abstand.

Weitere Merkmale der Erfindung ergeben sich aus der folgenden Beschreibung in Verbindung mit der Zeichnung und den einzelnen Patentansprüchen.

Ein Ausführungsbeispiel der Erfindung wird nachstehend anhand der Zeichnung im einzelnen beschrieben. Es zeigt:
**Fig.1** den in grösserem Massstab und im Schnitt dargestellten vorderen Augenabschnitt eines Lebewesens sowie eine mit einem auswechselbaren Werkzeug versehene Vorrichtung zum Zerkleinern und Entfernen des zerkleinerten Linsenkerns;
**Fig.2** die schematisch dargestellte und mit dem Werkzeug versehene Vorrichtung mit zugeordneter und wirkverbundener Saugpumpe und Infusionseinheit;
**Fig.3** ein in grösserem Massstab und räumlich dargestelltes Teilstück des Werkzeuges mit daran angeordnetem Schneidelement;
**Fig.4** das im Schnitt dargestellte Teilstück des Werkzeuges gemäss Fig.3 mit dem daran angeordneten Schneidelement; und
**Fig.5** das in Seitenansicht dargestellte Teilstück des Werkzeuges gemäss Fig.4.

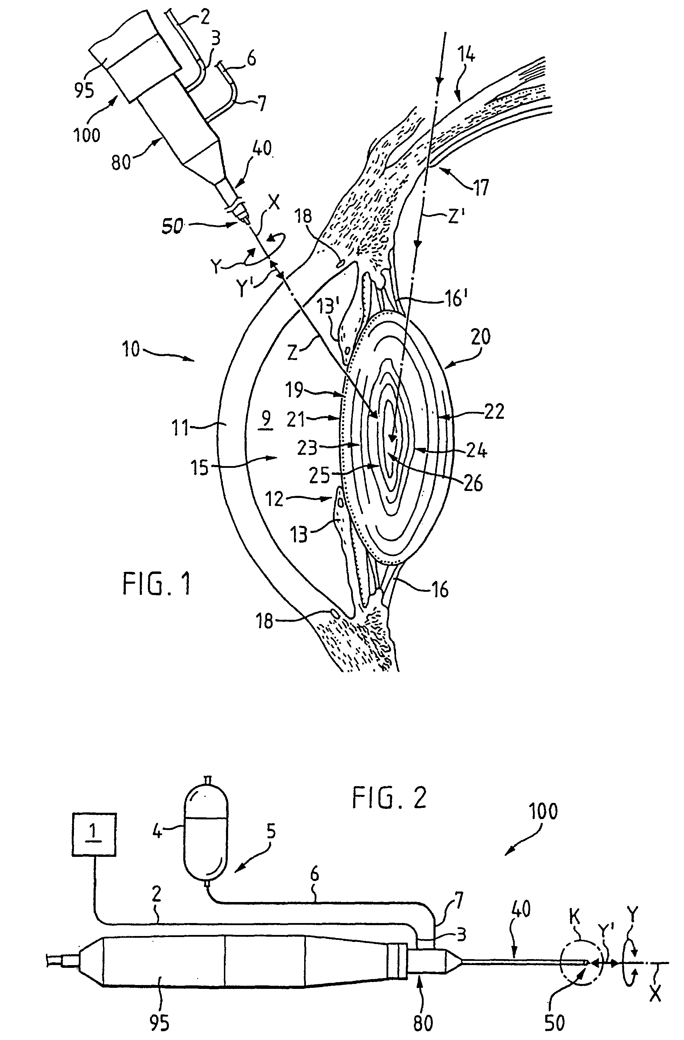

In Fig.1 ist ein in der Gesamtheit mit 10 bezeichneter Augenabschnitt eines Lebewesens in grösserem Massstab und im Schnitt dargestellt und man erkennt die Hornhaut 11 (Cornea), die Vorderkammer 9, die als Gesamtheit mit 12 bezeichnete Regenbogenhaut (Iris) mit den beiden zirkulären Bereichen 13 und 13', die Lederhaut 14 (Sklera), die Pupille 15, die Linse 20 (Okular) mit den Strahlenbändern 16,16' (Zonularfasern) sowie die gesamthaft mit 17 bezeichnete Pars plana und den mit 18 bezeichneten zirkulären Schlemmschen Kanal. Die als histologischer Schnitt dargestellte Linse 20 besteht aus mehreren, schematisch dargestellten Diskontinuitätszonen, welche sich, von aussen nach innen gesehen, wie folgt zusammensetzen: **a)** vordere und hintere Kapselhaut 19, **b)** nur vorne bis etwa zum Äquator reichende Epithelzellen 21 der Linse, **c)** vordere und hintere Abspaltungsfläche 22, **d)** vordere und hintere Alterskernzone 23, **e)** vordere und hintere äussere Embryonalkernzone 24, **f)** vordere und hintere innere Embryonalkernzone 25 und **g)** zentraler Interval 26.

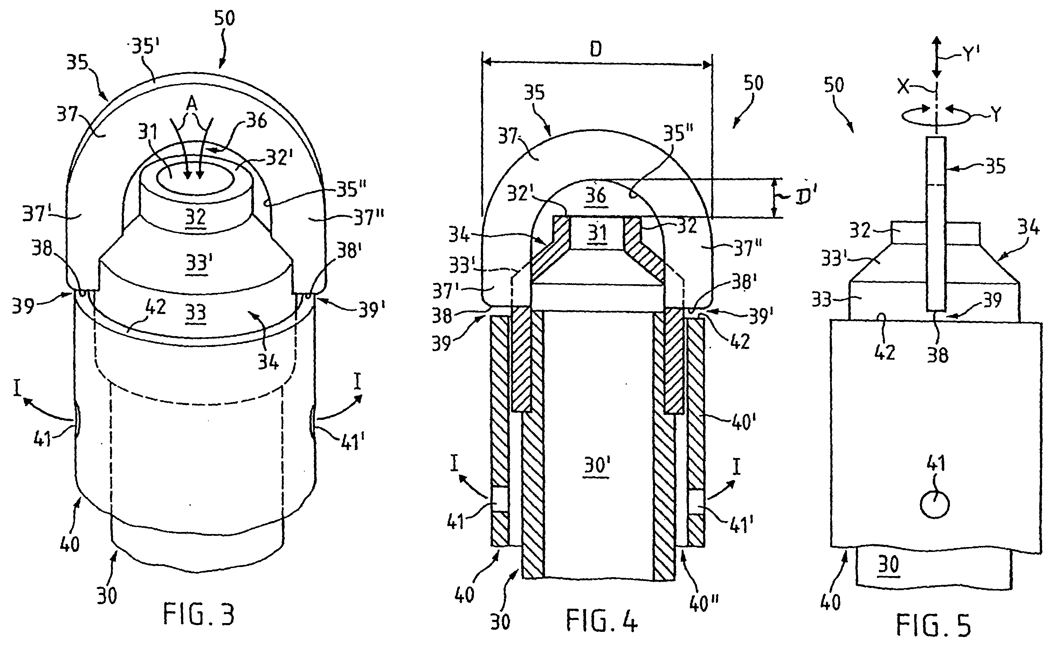

Weiterhin erkennt man in Fig.1 ein Teilstück einer schematisch dargestellten augenchirurgischen Vorrichtung 100, welche im wesentlichen ein als Griffstück ausgebildetes Gehäuse 95 und eine daran angeordnete Halterung 80 umfasst, welche zur Aufnahme eines in Fig.1 schematisch dargestellten Werkzeuges 50 zum Schneiden und/oder Zerkleinern des Linsenkerns im Auge ausgebildet ist. Das in der Halterung 80 angeordnete Werkzeug 50 steht mit nicht dargestellten, beispielsweise mit im Gehäuse 95 angeordneten Antriebsmitteln derart in Wirkverbindung, dass ein an dem Werkzeug 50 angeordnetes Schneidelement 35 (Fig.3 bis 5), wie in den Figuren 1 und 2 dargestellt, um eine Längsachse X in Pfeilrichtung Y rotierend angetrieben werden kann und/oder in Richtung der Längsachse X gemäss Pfeilrichtung Y' verschiebbar ist. Das Schneidelement 35 des Werkzeuges 50 kann aber auch zusätzlich zu der Rotation noch oszillierend angetrieben werden. Die nicht dargestellten Antriebsmittel sind vorzugsweise so ausgebildet, dass eine sogenannte Überlagerung der genannten Bewegungen und somit eine Kombination der rotierenden Bewegung mit der in axialer Richtung orientierten Bewegung möglich ist.

Zum Entfernen eines getrübten Linsengewebes oder Linsenkerns (Katarakt) wird in einer ersten Phase die Regenbogenhaut 12 entweder medikamentös oder durch entsprechend hakenförmig ausgebildete, nicht dargestellte Instrumente (Retractors) erweitert und in die Hornhaut 11 und Kapselhaut 19 eine etwa schlitzförmige Öffnung (nicht dargestellt) eingeschnitten. Anschliessend wird die Vorrichtung 100 mit dem Werkzeug 50, wie in Fig.1 schematisch mit Pfeilrichtung Z dargestellt, dem Augenabschnitt 10 zugeführt und in das Auge eingeführt. Hierbei wird das mit dem Schneidelement 35 versehene Werkzeug 50 durch die nicht dargestellte und bezeichnete, schlitzförmige Öffnung in die Linse 20 eingeführt. Das Werkzeug 50 umfasst im wesentlichen eine zentrisch in einem Führungsrohr 40 angeordnete und an dem einen freien Ende mit dem Schneidelement 35 versehene Rohrsonde 30.

Mittels des rotierend und/oder oszillierend angetriebenen Schneidelements 35 kann nunmehr der harte Linsenkern in Partikel zerkleinert und im wesentlichen in Form einer breiigen Masse oder dergleichen abgesaugt (Aspiration) und somit aus dem Kapselsack (nicht dargestellt) entfernt werden.

Wie in Fig.1 weiterhin schematisch dargestellt, kann die Einrichtung 100 jedoch auch in Pfeilrichtung Z' durch die Pars plana 17 in die Linse 20 eingeführt werden.

Fig.2 zeigt die in schematischer Ansicht dargestellte augenchirurgische Vorrichtung 100 mit dem als Handgriff ausgebildeten Gehäuse 95 und dem daran angeordneten Kopfstück 80. Die Vorrichtung 100 ist einerseits über eine Saugleitung 2 an eine Saugpumpe 1 angeschlossen und steht andererseits über eine Infusionsleitung 6 mit einem Infusionsbehälter 4 einer schematisch dargestellten Infusionseinheit 5 in Verbindung. Die Saugleitung 2 und Infusionsleitung 6 sind an entsprechend im Abstand zueinander an der Halterung 80 angeordneten Stutzen 3 beziehungsweise 7 angeschlossen. Weiterhin erkennt man in Fig.2 das schematisch dargestellte und an der Halterung 80 in nicht näher dargestellter Weise vorzugsweise auswechselbar angeordnete Werkzeug 50. Die vorstehend genannten Elemente 1 und 5 sowie 80 und 95 sind nicht Gegenstand der vorliegenden Erfindung und werden deshalb auch nicht näher beschrieben.

Die Figuren 3 bis 5 zeigen als erstes Ausführungsbeispiel das in Fig.2 durch einen Kreis K bezeichnete Teilstück des mit mindestens einem Schneidelement versehenen ersten Werkzeuges 50 in grösserem Massstab, wobei das Werkzeug 50 in Fig.3 in räumlicher Darstellung, in Fig.4 im Schnitt und in Fig.5 in Seitenansicht dargestellt ist. Das Werkzeug 50 umfasst im wesentlichen die koaxial in dem Führungsrohr 40 angeordnete Rohrsonde 30 mit dem daran angeordneten Schneidelement 35. Zwischen der Rohrsonde 30 und dem Führungsrohr 40 ist, wie in Fig.4 dargestellt, für die Infusion eines geeigneten Fluids ein kreisringförmiger sich in axialer Richtung erstreckender Spalt 40" vorgesehen. Das Werkzeug 50 wird nachstehend im einzelnen beschrieben.

Bei dem Werkzeug 50 ist an der zentrisch im Führungsrohr 40 angeordneten Rohrsonde 30 ein im wesentlichen trichterförmig ausgebildetes und mit einer Ansaugöffnung 31 versehenes Kopfstück 34 angeordnet, welches ein erstes zylindrisches Teilstück 33, ein daran angeformtes konisches Teilstück 33' sowie ein daran angeformtes zweites zylindrisches Teilstück 32 umfasst. Die Ansaugöffnung 31 des Kopfstücks 34 steht mit dem Innenraum 30' der Rohrsonde 30, welcher über die Saugleitung 2 mit der Saugpumpe 1 verbunden ist, in Verbindung. Das Kopfstück 34 ist mit dem ersten zylindrischen Teilstück 33 auf ein abgesetzt ausgebildetes Teilstück (nicht bezeichnet) der Rohrsonde 30 aufgesteckt und beispielsweise durch eine Schweissverbindung (Laserschweissung) befestigt (Fig.4). An den aus dem Führungsrohr 40 herausragenden Teilstücken 33, 33' des Kopfstücks 34 ist das im wesentlichen aus einem Bogenteil 37 und zwei daran angeformten Schenkeln 37' und 37" gebildete Schneidelement 35 angeordnet und befestigt. In dem Kopfstück 34 sind zwei diametral gegenüberliegend angeordnete Schlitze (nicht dargestellt) vorgesehen, in welchen die beiden im Abstand zueinander und achsparallel zur Längsachse X orientierten Schenkel 37',37'' des Schneidelementes 35 angeordnet und in nicht näher dargestellter Weise, beispielsweise durch Laserschweissung, befestigt sind. Das vorzugsweise einstückig ausgebildete und mit einer äusseren Schneidkante 35' versehene Schneidelement 35 ist mit den beiden Schenkeln 37',37'' derart an dem Kopfstück 34 befestigt, dass zwischen einer kreisringförmigen Fläche 32' des Teilstücks 32 und der kreisbogenförmigen Innenseite 35" des etwa hufeisenförmig ausgebildeten Schneidelements 35 ein Abstand 36 vorgeshen ist. Der in axialer Richtung zwischen der Kreistingförmigen Fläche 32' des Kopfstücks 34 und der Innen seite 35" des Bogenteils 37 vorgesehene Abstand 36 ist, wie in Fig.4 dargestellt, Kleiner als der quer dazu über die beiden Schenkel 37',37" des Bogenteils 37 gemessene äussere Abstand D. Zwischen der kreisringförmigen Oberkante 42 des Führungsrohres 40 und den zugeordneten Unterkanten 38,38' der beiden Schenkel 37',37'' des Schneidelements 35 ist jeweils ein Spalt 39,39' vorgesehen (Fig.3 bis 5).

Der in radialer Richtung über die beiden Schenkel 37',37" gemessene Abstand D (Aussenmass des Schneidelements 35) liegt etwa in der Grössenordnung von 1,6 mm und ist grösser als der in der Grössenordnung von 1,4 mm bis 1,5 mm liegende äussere Durchmesser (nicht bezeichnet) des Führungsrohres 40.

Fig.4 zeigt das in grösserem Massstab und in Schnittansicht dargestellte Werkzeug 50 und man erkennt das Führungsrohr 40, das koaxial darin angeordnete und an der Rohrsonde 30 befestigte Kopfstück 34 sowie das daran angeordnete und befestigte Schneidelement 35. In dem Führungsrohr 40 sind in axialer Richtung im Abstand zu der ringförmigen Oberkante 42 mindestens zwei diametral gegenüberliegende und die Wand 40' durchdringende Austrittsöffnungen 41 und 41' vorgesehen, welche mit dem zwischen der Rohrsonde 30 und dem Führungsrohr 40 bestehenden, kreisringförmigen Spalt 40" für die Zufuhr der Infusionsflüssigkeit in Verbindung stehen.

In Fig.5 ist das Werkzeug 50 in Ansicht dargestellt und man erkennt das Führungsrohr 40, die koaxial .darin angeordnete Rohrsonde 30 sowie das mit dem Schneidelement 35 versehene Kopfstück 34. Die Rohrsonde 30 sowie das Kopfstück 34 mit dem Schneidelement 35 sind, wie bereits in Verbindung mit Fig.2 erwähnt, mittels der im Gehäuse 95 angeordneten, nicht dargestellten Antriebsmittel einerseits um eine gemeinsame Achse X in Pfeilrichtung Y relativ zum feststehenden Führungsrohr 40 drehbar und andererseits gemäss Doppelpfeilrichtung Y' in axialer Richtung relativ zu der Oberkante 42 des Führungsrohres 40 bewegbar.

Zur Verdeutlichung der jeweiligen Abmessung des in den Figuren 3 bis 5 dargestellten Ausführungsbeispiels des Werkzeuges 50 für die augenchirurgische Vorrichtung 100 wird an dieser Stelle darauf hingewiesen, dass der Aussendurchmesser des Führungsrohres 40 etwa 1,5 mm und der Aussendurchmesser der Rohrsonde 30 etwa 1,0 mm beträgt.

Bei dem Werkzeug 50 ist das über das Schneidelement 35 gemessene Mass D, vorzugsweise grösser als der Durchmesser des Führungsrohres 40 (Fig.3; Fig.4) ausgebildet, wobei das lediglich in Fig.4 eingezeichnete Mass D, wie bereits vorstehend erwähnt, etwa in der Grössenordnung von 1,6 mm liegt. Eine weitere Verkleinerung der einzelnen Elemente ist jedoch anzustreben.

Mit dem in die Linse 20 (Fig.1 eingeführten sowie rotierend und vorzugsweise hochfrequent oszillierend angetriebenen Schneidelement 35 des Werkzeuges 50 besteht die Möglichkeit, dass die verhärtete und zu exzidierende Linsenkernstruktur zerkleinert wird und die Linsenpartikel durch Aspiration abgesaugt werden können. Eine Trübung der Linse wird bei gleichzeitiger Aspiration (A,A') der Linsenpartikel durch eine in den einzelnen Figuren mit I,I' bezeichnete Infusion eines geeigneten Fluids verhindert und dadurch während des mikrochirurgischen Eingriffs am Auge eine weitgehend uneingeschränkte Sicht bis zu dem in der Linse 20 (Fig.1) befindlichen Schneidelement oder dergleichen gewährleistet.

Durch die im wesentlichen kreisbogenförmige Ausbildung der am Schneidelement 35 angeordneten Schneidkante 35' ist beim operativen Eingriff eine unbeabsichtigte Verletzung des Kapselsacks weitgehend ausgeschlossen. Die besondere Ausgestaltung des Schneidelements 35 im vorderen Bereich gewährleistet, dass die während des operativen Eingriffs anfallenden Linsenteilchen (nicht dargestellt) in das Zentrum der Ansaugöffnung 31 gelangen und somit abgesaugt werden können. Zusammenhängende, zähe Gewebeteilchen oder grössere Linsenpartikel werden vorzugsweise mittels eines entsprechend ausgebildeten Werkzeuges zerkleinert und abgesaugt. Durch die in bezug auf den Aussendurchmesser des Führungsrohres 40 seitlich überstehenden Schenkel 37',37" des Schneidelements 35 wird auch ein Eindringen und Zerkleinern extrem harter Linsenkerne (Katarakts) gewährleistet.

Weiterhin wird darauf hingewiesen, dass vorzugsweise vor dem eigentlichen operativen Eingriff, d.h. vor dem Zerkleinern des harten Linsenkerns, die nicht bezeichnete Innenseite der Hornhaut 11 mit den Endothelzellen vor der Emulgation des Linsenkerns durch Injektion eines geeigneten Gels geschützt wird. Hierdurch wird ein zuverlässiger Schutz der Zellen während der gesamten Operationsdauer und somit etwaige Komplikationen mit abgestorbenen Endothelzellen weitgehend ausgeschlossen.

## Patentansprüche

1. Vorrichtung zum Zerkleinern und Entfernen des Linsenkerns aus dem Auge eines Lebewesens, mit einem als Handstück ausgebildeten Gehäuse (95), einer daran angeordneten Halterung (80) zur Aufnahme eines mit einer Infusionsleitung (6) in Verbindung stehenden Führungsrohres (40), einer koaxial darin angeordneten und relativ dazu um eine gemeinsame Längsachse (X) rotierend und/oder in axialer Richtung oszillierend antreibbaren Rohrsonde (30), welche an dem einen Ende mit einer Saugleitung (2) verbunden und an dem anderen Ende mit einem teilweise aus dem Führungsrohr (40) ragenden Kopfstück (34) und daran angeordnetem Schneidelement (35) versehen ist, **dadurch gekennzeichnet, daß** das kopfstück (34) mit einem ersten zylindrischen Teilstück (33), einem daran angeformten konischen Teilstück (33') sowie einem daran angeformten zweiten zylindrischen Teilstück (32) etwa trichterförmig ausgebildet und über eine Ansaugöffnung (31) mit dem Innenraum (30') der Rohrsonde (30) verbunden ist, daß das Schneidelement (35) mit einem Bogenteil (37) und daran angeordneter Schneidkante (35') die Ansaugöffnung (31) des Kopfstücks (34) etwa kreisbogenförmig überspannt, sowie mit zwei achsparallel zur Längsachse (X) angeformten Schenkeln (37',37") versehen ist, daß das Schneidelement (35) mittels der beiden Schenkel (37',37") derart an dem ersten zylindrischen Teilstück (33) des Kopfstücks (34) angeordnet und befestigt ist, dass ausgehend von der die Ansaugöffnung (31) des zweiten zylindrischen Teilstücks (32) kreisringförmig umgebenden Fläche (32') der bis zur Innenseite (35") des Bogenteils (37) in axialer Richtung orientierte Abstand (36) kleiner als der quer dazu über die beiden Schenkel (37',37") des Bogenteils (37) gemessene äussere Abstand (D) ist.

2. Vorrichtung nach Anspruch 1, **dadurch gekennzeichnet, dass** das Schneidelement (35) mit den beiden im Abstand zueinander angeordneten Schenkeln (37',37") derart in zwei diametral gegenüberliegenden Schlitzen des ersten zylindrischen Teilstücks (33) angeordnet und befestigt ist, dass zwischen der Unterkante (38;38') des einzelnen Schenkels (37';37") und der kreisringförmigen Oberfläche (42) des feststehenden Führungsrohres (40) jeweils ein Spalt (39;39') vorgesehen ist.

3. Vorrichtung nach Anspruch 1, **dadurch gekennzeichnet, dass** der über die beiden am Bogenteil (37) angeformten Schenkel (37';37") gemessene Abstand (D) grösser als der äussere Durchmesser des feststehenden Führungsrohres (40) ausgebildet ist, wobei der Abstand (D) des Schneidelements (35) in der Grössenordnung von 1,6 mm liegt.

4. Vorrichtung nach einem der Ansprüche 1 bis 3, **dadurch gekennzeichnet, dass** das Schneidelement (35) einstückig und etwa hufeisenförmig ausgebildet ist und das mit der äusseren Schneidkante (35') versehene Bogenteil (37) sowie die beiden gegenüberliegend und in parallelem Abstand zueinander daran angeformten Schenkel (37',37") umfasst.

5. Vorrichtung nach einem der Ansprüche 1 bis 4, **dadurch gekennzeichnet, dass** die Rohrsonde (30) zusammen mit dem daran befestigten Kopfstück (34) und dem Schneidelement (35) in Form einer aus der Rotations- und Oszillationsbewegung überlagerten und kombinierten Bewegung relativ zu dem feststehenden Führungsrohr (40) antreibbar ist.

## Claims

1. Device for crushing and removing the nucleus of the lens from the eye of a living organism, with a housing (95) constructed in the form of a hand piece, a mounting (80) disposed thereon to receive a guide tube (40) connecting with an infusion line (6), a tubular probe (30) disposed coaxially therein, which may be driven to rotate around a common longitudinal axis (X) relative thereto and/or oscillate in axial direction, said probe being connected at one end to a suction pipe (2) and at the other end to a head piece (34) projecting partially out of the guide tube (40) and being provided with a cutting element (35) disposed on said head piece, **characterised in that** the head piece (34) is constructed to be approximately funnel-shaped with a first cylindrical section (33), a conical section (33') moulded thereon as well as a second cylindrical section (32) moulded thereon and is connected to the interior (30') of the tubular probe (30) via a suction opening (31), **in that** the cutting element (35) spans the suction opening (31) of the head piece (34) with a curved portion (37) and a cutting edge (35') disposed thereon in an approximately circular arc shape, and is also provided with two legs (37', 37") moulded on axis-parallel to the longitudinal axis (X), **in that** the cutting element (35) is disposed and fastened on the first cylindrical section (33) of the head piece (34) by means of the two legs (37', 37") in such a way that starting from the area (32') surrounding the suction opening (31) of the second cylindrical section (32) in a circular ring shape, the spacing (36) oriented in axial direction to the inside (35") of the curved portion (37) is smaller than the outside spacing (D) measured transversely thereto over the two legs (37', 37") of the curved portion (37).

2. Device according to Claim 1, **characterised in that** the cutting element (35) with the two legs (37', 37") spaced from one another is disposed and fastened in two diametrically opposed slots of the first cylindrical section (33) in such a way that a gap (39; 39') is respectively provided between the lower edge (38; 38') of the individual leg (37'; 37") and the circular ring-shaped surface (42) of the fixed guide tube (40).

3. Device according to Claim 1, **characterised in that** the space (D) measured over the two legs (37'; 37") moulded onto the curved portion (37) is greater than the outside diameter of the fixed guide tube (40), the spacing (D) of the cutting element (35) being in the order of 1.6 mm.

4. Device according to one of Claims 1 to 3, **characterised in that** the cutting element (35) is constructed in one piece and in an approximately horseshoe shape and encloses the curved portion (37) provided with the outer cutting edge (35') as well as the two opposing legs (37', 37") spaced parallel to one another and moulded thereon.

5. Device according to one of Claims 1 to 4, **characterised in that** the tubular probe (30) together with the head piece (34) fastened thereto and the cutting element (35) may be driven relative to the fixed guide tube (40) in the form of a superposed and combined movement comprising the rotational and oscillatory movement.

## Revendications

1. Dispositif destiné à réduire et extraire le noyau du cristallin de l'il d'un être vivant, comportant un logement (95) conçu en tant que pièce à main, une fixation (80) disposée à son niveau et permettant de loger un tube de guidage (40) en relation avec une conduite de perfusion (6), une sonde tubulaire (30) disposée à l'intérieur de façon coaxiale et pouvant être entraînée de façon à pivoter par rapport à celle-ci autour d'un axe longitudinal conjoint (X) et/ou à osciller dans la direction axiale, laquelle sonde est reliée par une extrémité à une conduite d'aspiration (2) et est munie au niveau de l'autre extrémité d'un élément de tête (34) faisant partiellement saillie hors du tube de guidage (40), et d'un élément de coupe (35) disposé à son niveau, **caractérisé en ce que** l'élément de tête (34) est conçu approximativement en forme d'entonnoir par un premier élément cylindrique (33), un élément conique (33') façonné à son niveau et un second élément cylindrique (32) façonné à son niveau, et est relié par une ouverture d'aspiration (31) à l'espace interne (30') de la sonde tubulaire (30), **en ce que** l'élément de coupe (35) avec un élément courbe (37) et l'arête de coupe (35') disposée à son niveau sur-tend en arc de cerclé l'ouverture d'aspiration (31) de l'élément de tête (34) approximativement en forme de cercle, et est pourvu de deux branches (37',37") formées parallèlement à l'axe longitudinal (X), **en ce que** l'élément de coupe (35) est disposé et fixé au niveau du premier élément cylindrique (33) de l'élément de tête (34) au moyen des deux branches (37',37") de façon telle que, en partant de la surface (32') entourant en cercle l'ouverture d'aspiration (31) du second élément cylindrique (32), l'écartement orienté dans la direction axiale (36) jusqu'à la face interne (35") de l'élément arqué (37) est plus petit que l'écartement externe (D) mesuré transversalement à celui-ci via les deux branches (37',37") de l'élément arqué (37).

2. Dispositif selon la revendication 1, **caractérisé en ce que** l'élément de coupe (35) est disposé et fixé par les deux branches (37',37") disposées en formant un écart entre elles dans deux fentes diamétralement opposées du premier élément cylindrique (33) de façon telle qu'entre l'arête inférieure (38,38') d'une branche (37',37") et la surface circulaire (42) du tube de guidage (40) fixe soit prévue respectivement une fente (39;39').

3. Dispositif selon la revendication 1, **caractérisé en ce que** l'écartement (D) mesuré via les deux branches (37';37") formées au niveau de l'élément arqué (37) est supérieur au diamètre externe du tube de guidage (40), l'écartement (D) de l'élément de coupe (35) étant d'un ordre de grandeur de 1,6 mm.

4. Dispositif selon l'une des revendications 1 à 3, **caractérisé en ce que** l'élément de coupe (35) est conçu en une pièce et approximativement en forme de fer à cheval, et comprend l'élément arqué (37) équipé de l'arête de coupe externe (35') et les deux branches (37',37") formées à son niveau et placées à l'opposé selon un écartement parallèle.

5. Dispositif selon l'une des revendications 1 à 4, **caractérisé en ce que** la sonde tubulaire (30) et l'élément de tête (34) qui s'y raccorde et l'élément de coupe (35) peuvent être actionnés sous la forme d'un déplacement superposé et combiné de rotation et d'oscillation par rapport au tube de guidage (40) fixe.
